# FASCICULE DE BREVET EUROPEEN

(11) **EP 0 941 038 B1**
(45) Date de publication et mention de la délivrance du brevet: **07.07.2004**
(21) Numéro de dépôt: 98946528.1
(22) Date de dépôt: 29.09.1998
(51) Int. Cl.: A61F 2/06

(54) **ENSEMBLE MEDICAL D'INTERVENTION SUR UN CONDUIT ANATOMIQUE et COLLIER D'ETANCHEITE APPARTENANT A UN TEL ENSEMBLE**
Medizinische Einheit zur Behandlung eines Gefässes mit zugehörigem Dichtungsring
MEDICAL SET FOR INTERVENTION ON AN ANATOMICAL DUCT and appertaining SEALING RING

(30) Priorité: 01.10.1997 FR 9712231; 12.05.1998 FR 9805946; 15.06.1998 FR 9807527
(43) Date de publication de la demande: 15.09.1999
(73) Titulaire: B. Braun Celsa, 92100 Boulogne-Billancourt (FR)
(72) Inventeur: CHEVILLON, Gérard, F-92100 Montrouge (FR); NADAL, Guy, F-86000 Poitiers (FR); ANIDJAR, Samy, F-75013 Paris (FR)
(74) Mandataire: Lerner, François
(86) Numéro de dépôt international: PCT/FR1998/002087
(87) Numéro de publication internationale: WO 1999/016384

(56) Documents cités:
- EP-A- 0 579 523
- WO-A-90/15582
- WO-A-96/07371
- WO-A-97/19653

## Description

Le domaine de l'invention est celui des prothèses tubulaires, bifurquées ou droites, destinées à être implantées dans un conduit du corps humain ou animal pour y maintenir ou rétablir un passage.

En particulier, l'invention trouve son application dans l'utilisation d'une telle prothèse destinée à être placée à l'endroit d'un anévrisme vasculaire (en particulier de l'artère aorte), en vue d'isoler celui-ci tout en créant, à cet endroit, un substitut de la paroi du vaisseau pour que le sang puisse continuer à circuler.

On connaît déjà différentes prothèses (ou d'implants) destinées à être placées à l'endroit d'un anévrisme, qu'elles soient auto-expansibles (c'est-à-dire expansibles hors contrainte radiale interne, par "élasticité intrinsèque" ou par "mémoire de forme"), ou expansible à l'aide d'un moyen annexe créant une force interne radiale d'écartement (tel qu'un ballon). Ces prothèses comprennent en général une structure métallique comprenant au moins un "stent" (ou élargisseur) adapté pour prendre appui contre la paroi du vaisseau, au moins d'un côté de l'anévrisme, à un endroit dénommé "collet supérieur", et ce par création d'une force d'écartement de la prothèse vers la paroi du vaisseau.

Par collet supérieur (ou encore amont), on désigne la partie de la paroi du vaisseau située "au-dessus" de l'anévrisme et par collet inférieur, la partie de la paroi du vaisseau placée juste en aval de l'anévrisme (par rapport à la circulation sanguine).

Ces prothèses sont typiquement revêtues d'une gaine d'habillage, interne ou externe à la structure métallique et étanche (ou devenant étanche) au fluide-circulant. Elles peuvent être implantées dans le vaisseau à traiter, par exemple par voie endoluminale transcutanée (en particulier par la méthode dite "de SELDINGER"), avant de se déployer ou d'être déployées dans celui-ci et de se fixer à sa paroi. Ce type de prothèses est décrit en particulier dans FR-A-2 732 404, US-A-5 591229, US-A-5 330 500 ou EP-A-0 696 447.

Un problème rencontré est que ces prothèses sont en général insuffisamment adaptées aux conditions d'implantation, en particulier au collet (ou col) supérieur d'un anévrisme. La forme de ce collet (prise selon sa section ou longitudinalement) est rarement régulière (incurvée, à section variable, tapissée de divers agrégats cellulaires ou de creux) et très souvent différente d'un patient à un autre. Par conséquent, il y a une adéquation difficile entre le stent de la prothèse et la paroi du conduit. De plus, au cours du temps, le conduit a tendance à gonfler localement sous l'action de la prothèse et donc à se décoller d'elle. En outre, la prothèse peut parfois n'être pas bien fixée au conduit et risque alors de se déplacer intempestivement.

Certes, US-A-5 527 355 ou US-A-5 707 378 décrivent un ensemble médical destiné à être implanté dans un conduit anatomique et comprenant :
- une prothèse intraluminale comprenant une structure adaptée pour occuper un premier état radialement resserré pour son introduction dans le conduit, et un second état radialement déployé dans lequel la structure présente une forme générale de tube unique ou bifurqué pour prendre appui, au moins a son extrémité proximale supérieure, contre la paroi dudit conduit, à proximité de sa zone amont, et
- au moins un collier adapté pour être situé autour du conduit, en regard de l'extrémité proximale de la prothèse, pour la maintenir contre le conduit lorsque l'ensemble est implanté.

Le document WO 96/07371 décrit différentes sangles destinées à entourer un vaisseau sanguin et maintenues à une forme circulaire par un dispositif de fermeture. Il ne suggère pas de sangle déformable entre un état naturel courbe et un état déformé pour son implantation corporelle.

Les documents WO 97/19653 et EP-A 0 579 523 décrivent des prothèses pour conduits anatomiques, mais ne considèrent pas l'utilisation d'un collier ou autre dispositif autour de l'extérieur du conduit anatomique.

L'invention concerne, dans un ensemble à prothèse et collier, l'utilisation d'un collier déformable entre un état naturel de forme courbe, et un état déformé pour son implantation corporelle.

Plus précisément, l'invention concerne un ensemble médical selon la revendication 1 et un collier selon la revendication 9.

L'invention et sa mise en oeuvre apparaîtront encore plus clairement à l'aide de la description qui va suivre, relative à un traitement d'anévrisme et faite en référence aux dessins annexés dans lesquels :
- la figure 1 est une vue schématique en perspective d'un vaisseau atteint d'anévrisme et renfermant une prothèse anévrismale ainsi que deux colliers d'étanchéité schématiquement représentés,
- la figure 2 est une vue agrandie plus en détail d'un collier d'étanchéité,
- la figure 3 est une vue en coupe du collier selon la ligne III - III de la figure 2,
- la figure 4 représente schématiquement, en coupe partielle, un trocart de coelioscopie, ainsi qu'une pince de coelioscopie utilisables pour l'introduction d'un collier conforme à l'invention,
- la figure 5 montre l'utilisation du collier comme un "lac", alors manoeuvré par deux pinces de coelioscopie,
- la figure 6 montre le "lac" de la figure 5, en fin d'intervention, fixé étroitement de façon étanche autour du vaisseau (ici, collet supérieur),
- la figure 7 montre une manière d'introduire le collier de la figure 2 par l'intermédiaire d'un cathéter à poussoir,
- la figure 8 montre en coupe schématique un collier d'étanchéité en position autour d'une aorte qui adhère à des tissus corporels, le collier étant fixé à la prothèse aortique interne,
- la figure 9 est une variante de mise en place dans laquelle le conduit a pu être séparé des tissus corporels, le collier faisant alors tout le tour de ce conduit,
- la figure 10 représente, en vue perspective et conduit récepteur coupé, une variante de réalisation de l'ensemble collier/prothèse,
- et la figure 11 est une autre variante du même ensemble que celui de la figure 10.

Dans ce qui suit, on ne considérera que le cas du traitement d'un anévrisme de vaisseau, même si d'autres conduits anatomiques peuvent être concernés.

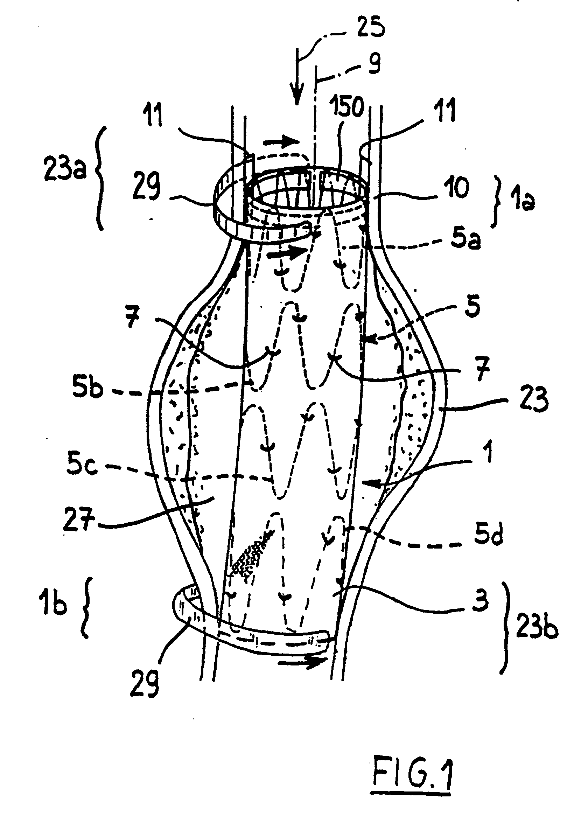

Sur la figure 1 tout d'abord, est représentée une prothèse "traditionnelle" pour anévrisme, 1.

Cet implant comprend une gaine tubulaire 3 flexible "usuelle", typiquement en tissu (par exemple, Dacron, marque déposée) et adaptée pour canaliser le sang à travers elle.

Une armature constituée d'une structure en stents repérée dans son ensemble 5 assure une tenue mécanique à la gaine 3, en étant liée à elle par un ou plusieurs fils de suture chirurgicale 7.

La structure 5 peut occuper deux états:
- un premier état de diamètre restreint (non représenté), pour l'introduction de l'implant à l'intérieur du vaisseau récepteur (en utilisant typiquement la méthode de "SELDINGER"),
- ou un second état de plus fort diamètre (voir figure 1) dans lequel l'implant est au moins localement sensiblement appliqué contre la paroi interne du vaisseau.

La structure en stent 5 peut être constituée de stent(s) autoexpansible(s), typiquement en métal tel de l'acier inoxydable, ou encore être expansible(s) par l'intermédiaire d'un ballon gonflable (non représenté). Une réalisation en matériau à mémoire de forme, en particulier à base d'un alliage nickel/titane (Nitinol, marque déposée) est aussi envisageable.

Dans l'exemple de la figure 1, la structure en stents 5 est échelonnée sur plusieurs étages, en l'espèce 5a, 5b, 5c, 5d. Chaque étage est constitué d'un fil métallique ondulé (tel qu'un zigzag) refermé sur lui-même pour former un anneau cylindrique (ou tube ajouré) de diamètre variable.

Les étages 5a et 5d sont situés respectivement vers les extrémités libres amont (par rapport au flux sanguin), 1a, et aval, 1b, de l'implant.

La structure 5 peut être disposée à l'intérieur ou à l'extérieur du manchon 3 qui est lui-même resserré ou radialement déployé (par rapport à l'axe 9 de l'implant), en fonction de l'état radial de la structure 5.

A noter qu'une forme autre qu'en fil(s) ondulé(s) pourrait être retenue pour la structure 5.

Pour sa fixation au vaisseau 10 après avoir été positionné à l'intérieur, l'implant 1 peut comprendre des crochets métalliques d'ancrage tels que 11, présentant une pointe adaptée pour pénétrer dans la paroi du vaisseau. Sur la figure 1, ils dépassent de l'extrémité amont 1a et sont liés à l'étage 5a.

En complément ou en substitution à de tels crochets, on conseille toutefois l'utilisation d'autres moyens de fixation associés à des moyens complémentaires d'étanchéité.

Comme décrit dans FR 9716625, déposé le 29 Décembre 1997, il s'agit de moyens de fixation qui traversent toute la paroi du vaisseau 10, et/ou qui vont s'y fixer depuis la paroi extérieure du vaisseau, tels que des rivets (au contraire des crochets 11 qui ne sont pas censés traverser cette paroi et qui s'accrochent depuis l'intérieur du vaisseau).

Une autre technique efficace de fixation peut consister à suturer (par un ou plusieurs files) de suture chirurgicale connus) l'implant au vaisseau, a priori à partir de l'extérieur du vaisseau, en utilisant alors une technique de coelioscopie extra-vasculaire.

Quels que soient ces moyens de fixation transvasculaire, ceux-ci seront présents de préférence tant à l'extrémité amont 23a qu'à l'extrémité aval 23b de l'anévrisme schématisé 23 sur la figure 1, sauf si l'on utilise la prothèse de FR-A-2 748 198 dont la gaine est, à son extrémité "aval", dépourvue de stent et est, là, anastomosée à la paroi en regard du conduit

Ces moyens de fixation sont associés à des moyens de renfort extérieur/étanchéité 29 pour empêcher le flux sanguin (flèche 25 pour le flux principal), de passer encore dans la poche anévrismale 27 (directement ou par "reflux" via des vaisseaux collatéraux) et aux collets du vaisseau de gonfler.

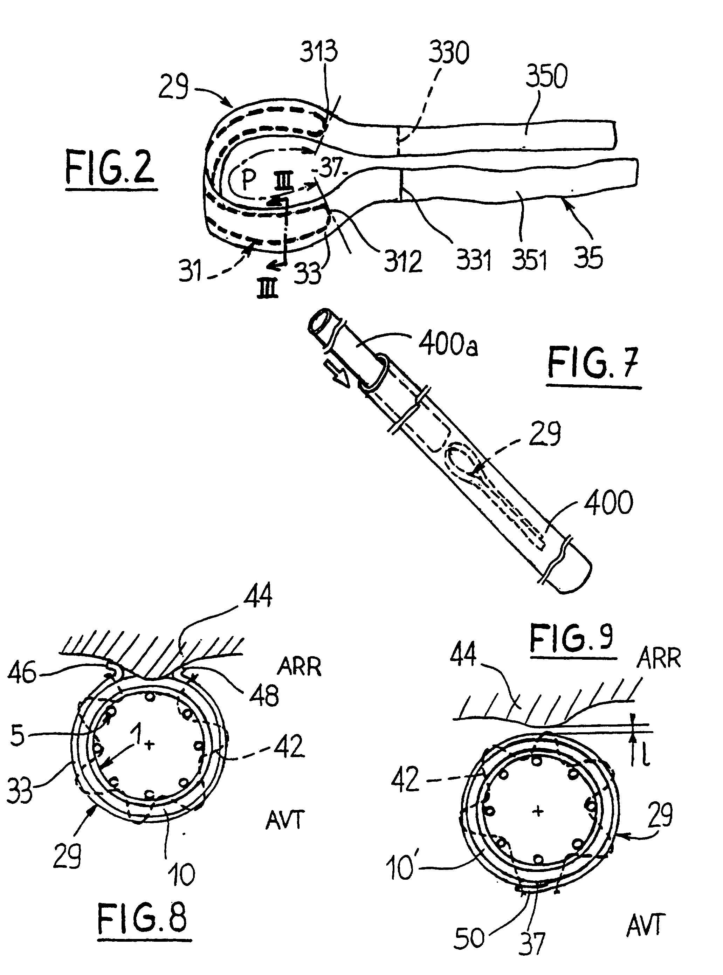

Sur la figure 2, le moyen d'étanchéité 29 représenté comprend une âme mécaniquement structurante 31 ayant la forme d'un anneau ou d'un collier fendu (non totalement fermé sur lui-même), naturellement arqué et présentant un effet ressort.

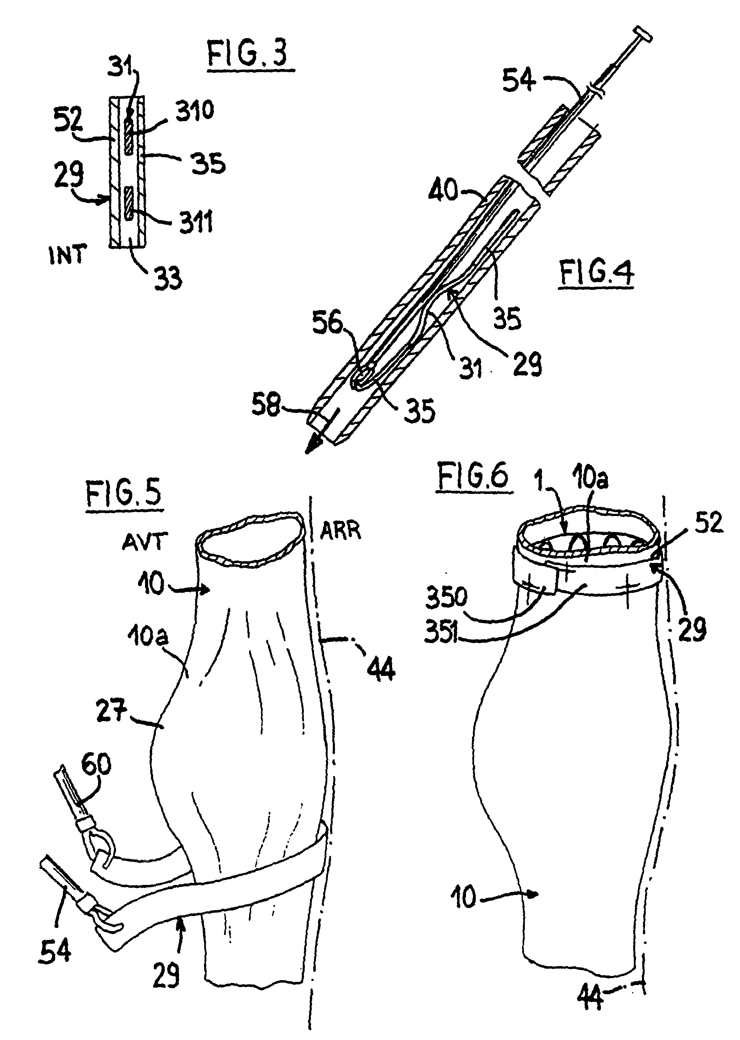

Comme on peut le voir encore plus précisément sur la figure 3, l'âme 31 peut en particulier comprendre deux lames métalliques 310, 311, sensiblement parallèles entre elles et écartées l'une de l'autre suivant le périmètre de l'anneau 31, pour définir entre elles une zone favorable de fixation du collier à la prothèse. Ces lames sont réunies ensemble, à leurs extrémités libres, en 310 et 311 (figure 2). Le diamètre "nominal" de l'anneau est de préférence égal ou légèrement inférieur à celui du conduit 10 pour le serrer naturellement très légèrement et l'empêcher de gonfler, malgré la poussée radiale interne de l'implant 1.

Cette armature annulaire ouverte 31 est revêtue d'une couche de protection en matière auto-obturante 33.

Ainsi, la lame ressort 31 n'est pas agressive pour le corps du patient.

A noter qu'à la place de métal (qui présente l'avantage d'être radio-opaque), la lame 31 pourrait être réalisée en matière plastique de qualité ressort telle qu'en polychlorure de vinyle (PVC) ou en polycarbonate. En tant que métal, on peut prévoir un acier inoxydable (Phynox, en particulier, marque déposée), ou un alliage à mémoire de forme tel que du Nitinol (marque déposée).

La matière du revêtement 33 peut être une silicone biocompatible.

Sur sa surface intérieure, l'anneau ainsi constitué comprend également une lanière 35 doublant l'âme ainsi revêtue.

La lanière 35 s'étend sur tout le périmètre de l'anneau revêtu et se prolonge au-delà du périmètre de l'âme 31, à l'endroit des prolongements 350, 351, comme le montre la figure 2.

Avantageusement, les prolongements 350 et 351 qui s'étendent de part et d'autre de l'âme 31 présenteront une longueur cumulée au moins sensiblement égale à la longueur du pourtour P de l'âme. A noter que cette longueur P sera typiquement inférieure à la circonférence extérieure du conduit à traiter, pour éviter d'en faire le tour.

La lanière 35 peut être réalisée en tissu (tel que du Dacron, marque déposée) voire en feutre biocompatible, ou en matière plastique très souple, de manière à pouvoir occuper la forme souhaitée par le praticien et être sécable par une pince de chirurgie, y compris si sa découpe doit s'effectuer dans le corps du patient.

Pour assurer une bonne qualité d'étanchéité pour des diamètres de vaisseaux différents, le revêtement auto-obturant de protection 33 se prolonge avantageusement au-delà des limites 312, 313, de l'âme 31, jusqu'aux marques repérées 330 et 331 sur la figure 2, c'est-à-dire sur une partie de la longueur des prolongements 350, 351, de la lanière.

Sur la figure 3, une couche supplémentaire 52 double également l'ensemble du collier 29. II s'agit d'une couche à caractéristique hémostatique, par exemple en feutre ou en moussse de Téflon (marque déposée), ou encore d'un tressage composite. Dans la technique, on dénomme couramment "pledget" une telle bande hémostatique destinée à réduire un écoulement sanguin lorsque cette bande est appliquée contre la fuite.

Bien entendu, on aura compris que la couche hémostatique 52 sera disposée sur la face intérieure (INT) du collier 29, pour être appliquée directement sur la paroi extérieure 10a du vaisseau 10 (figure 6).

Pour l'introduction du (de chaque) collier 29, on peut utiliser une gaine 40, couramment appelée "trocart" dans les domaines de l'endoscopie/coelioscopie/laparoscopie.

C'est d'ailleurs avec cette technique d'intervention peu invasive que l'on introduira, utilisera et fixera le (chaque) collier 29.

Ainsi, sur la figure 4, le trocart 40 renferme-t-il le collier 29 et une pince de coelioscopie 54 dont les mâchoires 56 tiennent entre elles une extrémité de la lanière 35 pour tirer vers l'intérieur du corps du patient (flèche 58) le collier représenté qui est alors dans une position déformée sensiblement à plat, avec sa partie annulaire fendue 31 complètement ouverte, sous contrainte élastique.

La coelioscopie étant une technique d'intervention connue, on ne la décrira pas ici, étant simplement noté que plusieurs voies d'abord corporel transcutané (avec ou sans dénudation de vaisseau) peuvent être réalisées, d'une part pour l'introduction du (des) collier(s) et d'autre part, pour la mise en place des outils chirurgicaux coelioscopiques nécessaires, comme par exemple une seconde pince repérée 60 sur la figure 5.

Sur la figure 5, le collier 29 est déjà en place autour du vaisseau 10 à traiter. Sous la traction de la pince 54, il a tout d'abord été extrait du trocart 40 (à l'intérieur du corps du patient) où une seconde pince (telle que la pince 60) l'attendait pour engager le collier si possible autour du vaisseau (étant précisé que cette manoeuvre de passage du collier autour du vaisseau peut être délicate, voire impossible, en particulier dans le cas de l'aorte, compte tenu de la présence des tissus d'attache à la colonne vertébrale, ici schématisée en 44).

Quoi qu'il en soit, s'il est possible de passer le collier 29 autour du conduit anatomique à traiter (voire d'un organe annexe), ce collier pourra être utilisé comme un "lac", dans le but de faciliter le travail du chirurgien. Pour cela, ce dernier pourra en particulier utiliser les pinces 54, 60 (figure 5), voire d'autres outils de coelioscopie, afin d'agir sur le conduit ou organe concerné, par l'intermédiaire des brins du collier dont la souplesse, associée éventuellement à la rigidité relative de l'armature 31, facilitera le mouvement recherché du conduit ou de l'organe.

Une fois terminée cette fonction de "moyen de manoeuvre du conduit ou de l'organe" assurée par le collier, ce dernier sera mis en place à l'endroit du collet supérieur (ou du collet inférieur) du vaisseau 10, à l'intérieur duquel la prothèse a déjà été installée.

Sur la figure 6, le collier et la prothèse sont d'ailleurs déjà en place, l'armature annulaire du collier s'étant d'elle-même refermée autour de la paroi extérieure 10a du vaisseau, après qu'on l'ait libérée de toute contrainte d'ouverture imposée par les pinces 54, 60.

A titre de variante, la couche à caractère hémostatique 52 pourrait être remplacée par un traitement de surface (enduction en particulier) avec un produit coagulant, voire un produit dispersé, ou noyé, dans le revêtement 33 et/ou la lanière 35.

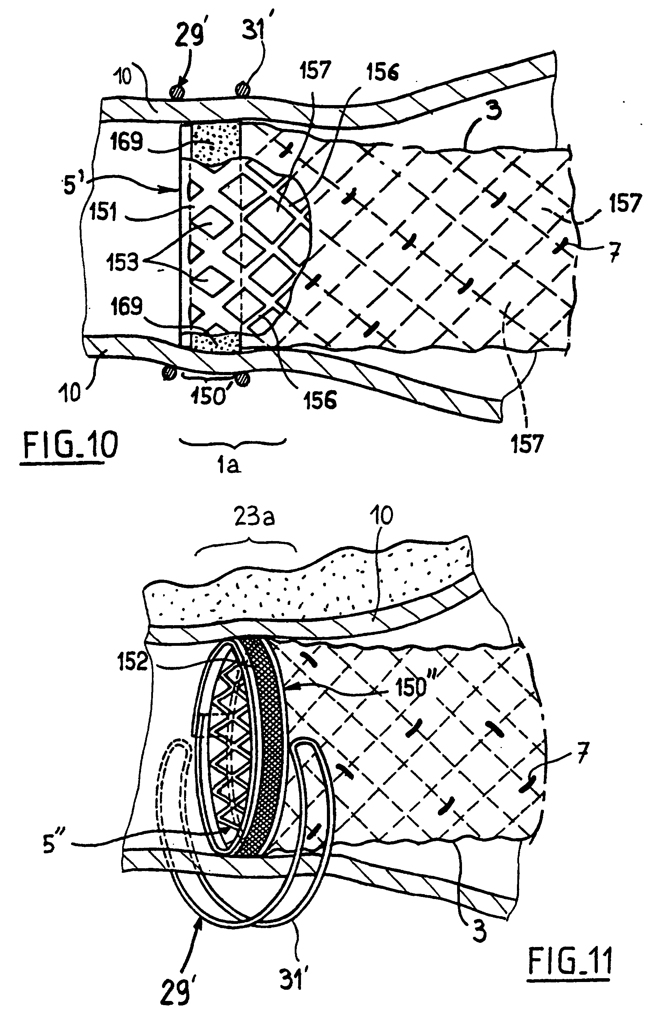

On notera également que plutôt que de déformer sensiblement à plat l'anneau 29, on pourrait prévoir de l'engager dans la gaine 400 en l'écrasant légèrement sur lui-même et en le poussant avec un poussoir flexible 400a, comme représenté sur la figure 7.

Une fois poussé hors de la gaine 400, le collier 29 pourra être saisi par les pinces du chirurgien, à l'intérieur du corps du patient.

Si la mise en place est à effectuer autour d'une aorte humaine fixée à l'arrière (ARR) aux tissus corporels 44, comme sur la figure 8, le chirurgien engagera le collier 29 par l'avant (repéré AVT), en ouvrant sa fente 37, en particulier par traction sur les portions de lanières 350, 351, puis en les relâchant, jusqu'à ce que l'anneau se referme autour du vaisseau.

Le praticien coupera ensuite à longueur lesdits prolongements de lanière (coupes repérées 46 et 48). De préférence, la découpe s'effectuera sur la partie du collier où s'étend le revêtement de protection auto-obturant 33, de manière à favoriser au mieux l'étanchéité.

Le praticien fixera alors ensemble le collier 29 et la prothèse interne 1, à travers le vaisseau intermédiaire 10. Sur la figure 8, on a schématisé, pour cette fixation, un lien de suture chirurgicale 42 qui traverse plusieurs fois, de part en part, à partir de l'extérieur du collier 29, l'ensemble collier/vaisseau/implant, si nécessaire en passant autour d'un ou plusieurs tronçons de stent 5. (De préférence, l'aiguille utilisée aura une section inférieure à celle du fil, pour favoriser l'étanchéité).

Bien entendu, plusieurs fils de suture peuvent être utilisés et le chemin du fil 42 de la figure 5 n'est qu'illustratif d'un principe général de suture bien connu des chirurgiens (vasculaires en particulier).

L'introduction des moyens de fixation (fils de suture avec pince de manoeuvre, "rivets" comme dans FR 97 16625) s'effectuera par des trocards de laparoscopie, et leur manoeuvre intracorporelle par des pinces.

Le repérage radio-opaque des lames 310, 311 ou de tout élément métallique rapporté sur le collier 29 définit au praticien l'endroit où il doit réaliser sa fixation.

Sur la figure 9, a été représenté un cas où le conduit (repéré 10') récepteur intérieurement de la prothèse pour anévrisme 1 est légèrement décollé des tissus corporels 44 (distance 1).

Dans ce cas de figure, il pourra être intéressant pour le praticien de situer la fente d'ouverture 37 du collier élastique 29 vers l'avant (AVT), plutôt que vers l'arrière (ARR) proche des tissus 44.

Ensuite, le praticien vient, comme déjà indiqué pour la figure 5, serrer ensemble de manière étanche au sang le collier, le conduit et la prothèse, à partir de l'extérieur du collier, et donc du conduit, par exemple à nouveau avec un fil de suture chirurgicale 42, ceci au moins à l'endroit du collet supérieur 23a, là où est reçu l'essentiel du flux sanguin 25.

Comme précédemment, le collier 29 (et en particulier sa bague fendue 31) sert d'appui, ou de renfort, extérieur pour la fixation et l'étanchéité.

A titre de variante de réalisation, la figure 10 montre que la prothèse peut à cet égard être associée à un renfort interne 150' prévu pour augmenter par l'intérieur la contrainte de pression exercée par la prothèse sur la paroi du vaisseau, à l'endroit de l'un et/ou l'autre des collets. Pour cela, l'anneau 150' est déformable radialement, et de préférence élastiquement

Ce renfort peut être structurellement indépendant de la prothèse (repère 150, figure 1), auquel cas, il sera de préférence implanté après celle-ci. Il peut toutefois, en alternative, être directement intégré à elle pour former une seule pièce (figures 10 et 11, repères 150' et 150" respectivement).

Dans le cas où le renfort est indépendant de la prothèse, sa structure peut être sensiblement la même que celle de l'anneau 31 (anneau fendu).

Dans le cas où le renfort est intégré à la prothèse, la structure de cette dernière peut être modifiée de telle façon que sa partie proximale (ou amont) 1a ainsi qu'éventuellement aval soit constituée par le renfort 150' (figure 10). Les éléments métalliques qui constituent là cette armature ont une section plus importante. S'il s'agit de fil(s) en zigzags, leur diamètre de fil est plus gros. Sur la figure 10, l'exemple retenu montre que sur le tronçon terminal 1a, les barrettes métalliques 151, qui limitent les cellules 153, sont plus larges que les barrettes 156 du reste de l'armature 5'. Les cellules 153 sont donc plus petites que celles 157 qui permettent l'expansion radiale du reste de l'armature.

Sur la figure 11, l'armature 5" est toujours interne et se présente encore, dans son état radialement déployé, comme un tube cylindrique de section (sensiblement) circulaire, troué à la manière d'une grille, à cellules en losange. La technique connue du "perçage laser" d'un tube métallique, à paroi pleine, a pu être utilisée pour cela. Le renfort 150" est maintenant disposé périphériquement à l'extérieur de l'implant, pour se plaquer directement contre le collet 23a. De petits trous (non représentés) peuvent être prévus pour le passage de liens de maintien entre ce renfort, le stent et la gaine 3.

En particulier pour éventuellement éviter d'avoir à munir l'implant de crochets d'ancrage, la surface externe 152 du renfort 150"peut être légèrement «abrasive» ou antidérapante.

S'il s'agit d'une pièce indépendante, la force d'écartement du renfort sera de préférence supérieure à celle d'un stent conventionnel.

Dans la variante des figures 10 et 11, on notera que l'anneau externe 29 est constitué uniquement d'un fil 31'plié pour présenter deux "cerclages" parallèles et espacés d'une distance constante sensiblement égale à la largeur du renfort. De cette façon, ce dernier vient, en position déployée de l'ensemble, s'insérer entre les deux cerclages, de façon à être bloqué en translation. Cette solution à cerclages peut aussi être appliquée à une bague à surface pleine, auquel cas lesdits cerclages sont proéminents vers l'intérieur de façon à réserver une zone annulaire pouvant recevoir ledit renfort

Il est également possible d'ajouter entre la prothèse et la paroi du vaisseau un tampon de mousse biocompatible pour, d'une part, éviter d'abîmer celle-ci et, d'autre part, améliorer encore le contact étroit, étanche, entre la prothèse et la paroi du vaisseau en "absorbant" les défauts de cette paroi. Ce tampon annulaire, schématisé en 169, sur la figure 10, recouvrira de préférence la prothèse, extérieurement, avant son implantation, à l'endroit de sa partie proximale et/ou distale.

En tant qu'alternative d'application, on pourrait envisager d'adapter l'ensemble présenté ci-avant pour l'implanter dans un autre conduit anatomique tel que la vésicule biliaire, voir à cet égard WO-A-97/09008 (dimensions, matière, ..., cf. p 8 à 11).

## Revendications

1. Ensemble médical destiné à être implanté, comprenant :
- une prothèse (1) introductible dans un conduit anatomique (10) lequel présente une zone amont (23a) par rapport au sens de circulation d'un fluide y cheminant, la prothèse, qui a un axe général et qui présente sensiblement selon cet axe deux extrémités libres, respectivement proximale (1a) et distale (1b), comprenant une structure adaptée pour occuper un premier état radialement resserré, pour son introduction dans le conduit, ou un second état radialement déployé dans lequel la structure présente une forme générale de tube unique ou bifurqué destiné à être placé sensiblement en appui, au moins vers son extrémité proximale (1a), contre la paroi du conduit (10), à proximité de sa zone amont, et
- au moins un collier (29, 31, 150, 150', 150") adapté pour être situé à l'extérieur du conduit au moins en regard de l'extrémité proximale (1a) de la prothèse (1) à l'endroit de ladite zone amont du conduit lorsque l'ensemble y est implanté,
**caractérisé en ce que** le collier (29, 31, 150, 150', 150") est déformable radialement au moins localement entre un premier état naturel, où il présente une forme courbe, et un second état déformé, pour son implantation corporelle, et la prothèse comporte un anneau interne de renfort (150, 150', 150") déformable radialement, pouvant être disposé en regard du collier, à l'intérieur du conduit.

2. Ensemble selon la revendication 1, **caractérisé en ce que** l'anneau interne de renfort (150', 150") est intégré à la structure de la prothèse (1) au moins vers son extrémité proximale (1a).

3. Ensemble selon la revendication 4, **caractérisé en ce que** l'anneau interne de renfort (150, 150") comprend une bague rapportée, déformable radialement et fendue.

4. Ensemble selon l'une quelconque des revendications précédentes, **caractérisé en ce que** ledit collier comprend une bague fendue (31) à disposer autour du conduit.

5. Ensemble selon la revendication 4, **caractérisé en ce que** la bague (31) est métallique.

6. Ensemble selon l'une des revendications 4 et 5, **caractérisé en ce que** la bague du collier externe (29) comprend deux cerclages (310, 311) sensiblement parallèles et espacés d'une distance telle que, dans l'état radialement déployé de la prothèse (1), l'anneau interne de renfort (150') se loge entre ces deux cerclages.

7. Ensemble selon l'une quelconque des revendications précédentes, **caractérisé en ce que** l'anneau interne de renfort (150") entoure la prothèse et présente une surface externe (152) abrasive et/ou antidérapante.

8. Ensemble selon l'une quelconque des revendications précédentes, **caractérisé en ce que** l'anneau de renfort s'étend à l'endroit d'une zone du collier où celui-ci présente une matière auto-obturante (33).

9. Collier destiné à être placé à l'extérieur d'un conduit anatomique (10) en regard d'un anneau de renfort d'une prothèse placé à l'intérieur de ce conduit, **caractérisé en ce qu'**il comprend :
- une âme (31) ayant une forme d'anneau fendu, naturellement courbe et à effet ressort,
- un revêtement (33) de protection de l'âme en une matière auto-obturante, et
- une lanière (35) sécable doublant l'âme ainsi revêtue, la lanière s'étendant au-delà du périmètre (P) de l'âme (31), et
le collier (29, 31, 150, 150', 150") est déformable radialement au moins localement entre un premier état naturel, où il présente une forme courbe, et un second état déformé, pour son implantation corporelle.

10. Collier selon la revendication 9, **caractérisé en ce que** l'âme (31) comprend deux lames (310, 311) métalliques, sensiblement parallèles entre elles et écartées l'une de l'autre.

11. Collier selon l'une quelconque des revendications 9 et 10, **caractérisé en ce que** le revêtement de protection (33) est constitué en un matériau sécable et s'étend au-delà du périmètre (P) de l'âme (31), le long de la lanière (35).

12. Collier selon l'une quelconque des revendications 9 à 11, **caractérisé en ce qu'**il présente une couche ou un revêtement hémostatique (52), pour réduire un écoulement sanguin, lorsque ledit conduit est un vaisseau contre lequel le collier (29) est appliqué.

## Patentansprüche

1. Medizinischer Aufbau, der zum Implantieren bestimmt ist, mit:
- einer in einen anatomischen Gang (10) einführbaren Prothese (1), der eine bezüglich der Zirkulationsrichtung eines dort fließenden Fluids aufstromige Zone (23a) aufweist, wobei die Prothese, welche eine allgemeine Achse hat und im wesentlichen entlang dieser Achse zwei freie Enden, proximal (1a) bzw. distal (1b) aufweist, eine Struktur aufweist, die geeignet ist, einen ersten radial eingeengten Zustand für ihre Einführung in den Gang oder einen zweiten radial entfalteten Zustand einzunehmen, in welchem die Struktur eine allgemeine Form eines einzigen oder gegabelten Rohres aufweist, das dazu bestimmt ist, im wesentlichen unter Abstützung mindestens gegen ihr proximales Ende (1a) gegen die Wand des Gangs (10) nahe seiner aufstromigen Zone angebracht zu werden, und
- mindestens einem Reif (29, 31, 150, 150', 150"), der geeignet ist, außerhalb des Ganges mindestens gegenüber dem proximalen Ende (1a) der Prothese (1) an der Stelle der aufstromigen Zone des Ganges zu liegen, wenn der Aufbau dort implantiert ist,
**dadurch gekennzeichnet, daß** der Reif (29, 31, 150, 150', 150") radial mindestens örtlich zwischen einem ersten natürlichen Zustand, in dem er eine gekrümmte Form aufweist, und einem zweiten verformten Zustand für seine körperliche Implantation verformbar ist, und die Prothese einen radial verformbaren inneren Verstärkungsring (150, 150', 150") aufweist, der gegenüber dem Reif im Inneren des Ganges angeordnet werden kann.

2. Aufbau nach Anspruch 1, **dadurch gekennzeichnet, daß** der innere Verstärkungsring (150', 150") in die Struktur der Prothese (1) wenigstens zu ihrem proximalen Ende (1a) hin integriert ist.

3. Aufbau nach Anspruch 4, **dadurch gekennzeichnet, daß** der innere Verstärkungsring (150, 150") einen angesetzten Reifen aufweist, der radial verformbar und geschlitzt ist.

4. Aufbau nach einem der vorhergehenden Ansprüche, **dadurch gekennzeichnet, daß** der Reif einen geschlitzten Reifen (31) zur Anordnung um den Gang aufweist.

5. Aufbau nach Anspruch 4, **dadurch gekennzeichnet, daß** der Reifen (31) metallisch ist.

6. Aufbau nach einem der Ansprüche 4 und 5, **dadurch gekennzeichnet, daß** der Reifen des äußeren Reifs (29) zwei im wesentlichen parallele Umreifungsbänder (310, 311) aufweist, die mit einem solchen Abstand voneinander entfernt sind, daß sich der innere Verstärkungsring (150') in radial entfaltetem Zustand der Prothese (1) zwischen diese zwei Umreifungsbänder einfügt.

7. Aufbau nach einem der vorhergehenden Ansprüche, **dadurch gekennzeichnet, daß** der innere Verstärkungsring (150") die Prothese umgibt und eine äußere abrasive und/oder Antirutsch-Oberfläche (152) aufweist.

8. Aufbau nach einem der vorhergehenden Ansprüche, **dadurch gekennzeichnet, daß** sich der Verstärkungsring an der Stelle einer Zone des Reifs, wo dieser ein selbstverschließendes Material (33) aufweist, erstreckt.

9. Reif, der dazu bestimmt ist, außerhalb eines anatomischen Ganges (10) gegenüber einem Verstärkungsring einer Prothese angebracht zu werden, der im Inneren dieses Ganges angeorndet ist, **dadurch gekennzeichnet, daß** er aufweist:
- einen Kern (31), der eine geschlitzte Ringform, natürlich gekrümmt und mit Federwirkung, hat,
- eine Schutzverkleidung (33) des Kerns aus einem selbstverschließenden Material, und
- einen schneidbaren Riemen (35), welcher den so verkleideten Kern verdoppelt, wobei sich der Riemen über den Durchmesser P des Kerns (31) erstreckt, und
- der Reif (29, 31, 150, 150', 150") radial mindestens örtlich zwischen einem ersten natürlichen Zustand, wo er eine gekrümmte Form aufweist, und einem zweiten für seine körperliche Implantation verformten Zustand verformbar ist.

10. Reif nach Anspruch 9, **dadurch gekennzeichnet, daß** der Kern (31) zwei metallische Blätter (310, 311) aufweist, die im wesentlichen zueinander parallel und voneinander im Abstand gehalten sind.

11. Reif nach einem der Ansprüche 9 und 10, **dadurch gekennzeichnet, daß** die Schutzverkleidung (33) aus einem schneidbaren Material besteht und sich über den Umfang (P) des Kerns (31) entlang dem Riemen (35) erstreckt.

12. Reif nach einem der Ansprüche 9 bis 11, **dadurch gekennzeichnet, daß** er eine hämostatische Schicht oder Verkleidung (52) aufweist, um einen Blutabfluß zu verringern, wenn der Gang ein Gefäß ist, gegen das der Reif (29) aufgebracht wird.

## Claims

1. A medical set adapted to be implanted, comprising :
a prosthesis (1) adapted to be introduced in an anatomical duct (10) including an upstream area (23a) in relation with the circulation direction of flowing fluid, said prosthesis, having a general axis and having along said axis two free ends proximal (1a) and distal (1b) respectively, comprising a structure adapted to have a first radially restricted state, to be inserted in the duct, or a second radially expanded state in which said structure said structure has a general single or bifurcate tubular configuration adapted to be disposed, at least near its proximal end (1a), substantially against wall of said duct (10), near its upstream area, and
at least a strap (29, 31, 150, 150', 150") adapted to be disposed outside of said duct at least opposite said proximal end (1a) of said prosthesis (1) where is said duct upstream area when said set is implanted,
**characterized in that** said strap (29, 31, 150, 150', 150") is at least locally radially deformable between a first natural state, in which it has a curved form, and a second distorted state, for corporal implantation, and said prosthesis includes an internal reinforcing, radially deformable ring (150, 150', 150") which may be disposed opposite said strap, inside said duct.

2. The medical set according to claim 1, **characterized in that** said internal reinforcing ring (150', 150") is integrated to the structure of prothesis (1) at least near its distal end (1a).

3. The medical set according to claim 4, **characterized in that** said internal reinforcing ring (150, 150") includes an inserted split radially deformable ring.

4. The medical set according to any preceding claim, **characterized in that** said strap includes a split ring (31) to be disposed around said duct.

5. The medical set according to claim 4, **characterized in that** said ring (31) is metallic.

6. The medical set according to claims 4 and 5, **characterized in that** said external strap ring (29) has two hoopings (310, 311) which are substantially parallel and spaced one from the other such that, in said radially expanded state of said prosthesis (1), said internal reinforcing ring (150') is received between two said hoopings.

7. The medical set according to any preceding claim, **characterized in that** said internal reinforcing ring (150") surrounds said prosthesis and presents an external abrasive and/or non-slipping surface (152).

8. The medical set according to any preceding claim, **characterized in that** said internal reinforcing ring extends at a strap area where that presents a self-obturing material (33).

9. A strap adapted to be disposed outside an anatomical duct (10) opposite to a reinforcing ring of a prothesis, disposed inside said duct, **characterized in that** it includes :
- a core (31) having a naturally curved springy split ring form,
- a self-obturing material coating (33) for protecting said core, and
- a divisible lining (35) for lining said coated core, said lining extending beyond perimeter (P) of said core (31), and
said strap (29, 31, 150, 150', 150") is radially deformable at least locally between a first natural state, in which it has a curved form, and a second distorted state, for corporal implantation.

10. The strap according to claim 9, **characterized in that** said core (31) includes two metallic blades (310, 311) substantially parallel and separated one from the other.

11. The strap according to any of claims 9 and 10, **characterized in that** said protecting coating (33) is constituted by a divisible material and extends beyond perimeter (P) of said core (31), along said lining (35).

12. The strap according to any of claims 9 to 11, **characterized in that** it presents a hemostatic layer or coating (52) for reducing blood flowing when said duct is a vessel against which is applied said strap (29).
